Europäisches Patentamt

European Patent Office

Office européen des brevets

(11) Veröffentlichungsnummer: **0 336 147**

**A2**

(12) **EUROPÄISCHE PATENTANMELDUNG**

(21) Anmeldenummer: 89104496.8

(22) Anmeldetag: 14.03.89

(51) Int. Cl.4: **C07D 231/12 , C07C 101/453 , C07C 69/738 , C07C 69/612 , C07C 69/65 , C07C 67/08 , A61K 31/415 , A61K 31/25**

(30) Priorität: 31.03.88 DE 3811118

(43) Veröffentlichungstag der Anmeldung:
11.10.89 Patentblatt 89/41

(84) Benannte Vertragsstaaten:
AT BE CH DE ES FR GB GR IT LI LU NL SE

(71) Anmelder: Merckle GmbH
Dr.-Georg-Spohn-Strasse 7
D-7902 Blaubeuren(DE)

(72) Erfinder: Metz, Gunter, Dr.
Auf dem Rucken 29
D-7902 Blaubeuren(DE)
Erfinder: Räuchle, Kurt, Dr.
Forstweg 16
D-7901 Blaubeuren-Sonderbuch(DE)
Erfinder: Erdmann, Manfred
Josef-Freundorfer Strasse 2A
D-7910 Neu-Ulm 8(DE)

(74) Vertreter: Lederer, Franz, Dr. et al
Van der Werth, Lederer & Riederer
Patentanwälte Lucile-Grahn-Strasse 22
D-8000 München 80(DE)

(54) Diethylenglykolmonoester, Verfahren zu ihrer Herstellung und ihre Verwendung als Arzneimittel.

(57) Diethylenglykolmonoester von antirheumatisch wirksamen Säuren wie z.B. Lonazolac und Diclofenac u.ä. sind neue Verbindungen und besitzen gegenüber den Säure-Grundkörpern bessere entzündungshemmende Eigenschaften. Sie können durch direkte Veresterung ohne Wasserauskreisung hergestellt werden und sind zur Bekämpfung von Krankheiten des rheumatischen Formenkreises geeignet.

EP 0 336 147 A2

## Diethylenglykolmonoester, Verfahren zu ihrer Herstellung und ihre Verwendung als Arzneimittel

Vorliegende Erfindung betrifft Diethylenglykolmonoester von antirheumatisch wirksamen Säuren der Gruppe bestehend aus

(a) 3-(4-Chlorphenyl)-1-phenyl-1H-pyrazol-4-essigsäure (Lonazolac)

(b) 2-[(2,6-Dichlorphenyl)amino]-phenylessigsäure (Diclofenac)

(c) 2-(2-Fluor-4-biphenylyl)-propionsäure (Flurbiprofen)

(d) 2-(4-Isobutylphenyl)-propionsäure (Ibuprofen)

(e) 2-(3-Benzoylphenyl)-propionsäure (Ketoprofen)

sowie im Falle von c) bis e) deren optisch aktiven, rechts-und linksdrehenden Isomere.

Die Herstellung des Diethylenglykolmonoesters der Flufenaminsäure durch Umsetzung von 2-(2-Chlorethoxy)ethanol mit dem Kaliumsalz von Flufenaminsäure ist bekannt (DE-PS 19 39 112). Ebenso ist bekannt, daß unter saurer Katalyse und Auskreisen des Reaktionswassers bei höheren Temperaturen (100° bis 180°) Flufenaminsäure mit 2-(2-Hydroxyethoxy)ethanol (Diethylenglykol) direkt umgesetzt werden kann (DE-PS 28 34 167) oder durch Umesterung niedermolekularer Ester mit Diethylenglykol unter basischen Bedingungen erhältlich ist (DE-PS 28 34 168). Die beiden letztgenannten Verfahren sind auch zur Herstellung der Diethylenglykolmonoester der Säuren (a) bis (e) geeignet, wobei lediglich beim Einsatz von Diclofenac wegen dessen ausgeprägter Tendenz zum Ringschluß die Reaktionstemperatur auf max. 140°C gesenkt werden muß. Ein besonders schonendes Verfahren zur Herstellung reiner Ester, das auch im Falle der Säuren (a) bis (e) Ausbeuten von 60-75 % ergab, ist die mit Carbonyldimidazol katalysierte direkte Umsetzung bei Raumtemperatur. Dieses Verfahren ist jedoch technisch aus Kostengründen schlecht nutzbar.

Wie überraschend gefunden wurde, kann die Veresterung der Säuren (a) bis (e) auch unter saurer Katalyse ohne Auskreisen des Reaktionswassers bei mittleren Temperaturen und kurzen Reaktionszeiten durchgeführt werden, wenn je Mol Säure 8 bis 15 Mol Diethylenglykol, vorzugsweise 10,5 bis 12 Mol, eingesetzt werden. Geeignete saure Katalysatoren sind Lewis Säuren, Zinkchlorid, Schwefelsäure, Phosphorsäure, Polyphosphorsäure, Benzolsulfonsäure, p-Toluolsulfonsäure oder Gemische aus Phosphorsäure und p-Toluolsulfonsäure, bei molaren Verhältnissen von 0,1 bis 1,0 Mol Katalysator je Mol Säure. Die Reaktionstemperatur beträgt 60° bis 120° bevorzugt 80 bis 100° bei Reaktionszeiten von 1 bis 5 Stunden.

Es überrascht besonders, daß das Verbleiben des Reaktionswassers im Ansatz eine derart hohe Esterausbeute erlaubt, da nach gängiger Lehrmeinung, auf der auch die DE-PS 28 34 167 aufbaut, ein Verschieben des Reaktionsgleichgewichtes zugunsten des Esters Entfernung des Reaktionswassers, d.h. azeotropes Auskreisen erfordert.

Nach der Reaktion wird der Überschuß an Diethylenglykol nach Neutralisation mit gegebenenfalls in fester Form zugesetzten Alkali- oder Erdalkalihydroxiden oder -carbonaten durch Destillation entfernt oder vorteilhaft durch Zugabe von Wasser, wobei sich der Rohester abscheidet oder durch Extraktion mit geeigneten Lösungsmitteln gewonnen wird. Der erhaltene Rohester wird entweder in einer zweistufigen Vakuum-Kurzwegdestillationsanlage destilliert oder mittels präparativer Mitteldruck-Flüssigkeitschromatographie über übliche Sorbentien, vorzugsweise Kieselgel, gereinigt. Drucke von 10-20 bar sind geeignet.

Die erfindungsgemäßen Diethylenglykolmonoester besitzen entzündungshemmende Eigenschaften. Sie können in Arzneimitteln verwendet werden, wobei Konzentration und Dosierung denen der bekannten Säure-Grundkörpern entsprechen.

Die entzündungshemmenden Eigenschaften der neuen Ester wurden im Vergleich zum jeweiligen Standard im Pfotenoedemtest der Ratte gemessen. Bei oraler Verabfolgung erhielten die Tiere (n = 5 je Gruppe) die Substanz 1 Stunde vor der intraplantaren Injektion von Carrageenin (1 % Suspension, 0,1 ml). Der Substanzeffekt wurde 3 Stunden nach der Injektion bestimmt. Für die dermale Applikation erhielten die Tiere (n = 10 je Gruppe) die Substanz gelöst in einem konstanten Volumen eines verträglichen organischen Lösungsmittels. Die Lösung wurde 0,5 Stunden vor und 2,5 Stunden nach intraplantarer Injektion von Kaolin (10 % Suspension, 0,1 ml) auf die gleiche Pfote aufgetragen. Der Meßwert wurde 4 Stunden nach der Injektion bestimmt. Der Hemmeffekt bezieht sich auf den jeweiligen Wert der unbehandelten Kontrollgruppe, dem jeweils das Vehikel verabreicht wurde. Die Ergebnisse sind in Tabelle 1 zusammengefaßt.

Tabelle 1

| Substanz | orale Applikation | | dermale Applikation | |
|---|---|---|---|---|
| | Dosis mg/kg | % Hemmung | Dosis mg/kg | % Hemmung |
| Diclofenac | 100 | 38 | 10 | 36 |
| Diclofenac Ester | 50 | 39 | 13* | 47 |
| Flurbiprofen | 50 | 40 | 10 | 28 |
| Flurbiprofen Ester | 10 | 38 | 13,6* | 41 |
| Ibuprofen | 100 | 41 | 10 | 8 |
| Ibuprofen Ester | 50 | 42 | 14,3* | 29 |
| Ketoprofen | 5 | 38 | 10 | 33 |
| Ketoprofen Ester | 2,5 | 36 | 13,5* | 45 |
| Lonazolac | 25 | 38 | 10 | 3 |
| Lonazolac Ester | 25 | 49 | 12,8* | 26 |

\* die Dosierung entspricht jeweils 10 mg/kg des Standards

Wie die Ergebnisse zeigen, sind die neuen Ester der jeweiligen Säure als Standard wirkungsmäßig überlegen. Im Vergleich zum Standard wird nach oraler Verabfolgung der gleiche Hemmeffekt mit wesentlich reduzierter Dosis der Ester erzielt oder ist stärker ausgeprägt (Lonazolac). Bei dermaler Anwendung sind innerhalb des Meßzeitraumes die Ester bei äquimolarer Dosierung deutlich überlegen.

Die erfindungsgemäßen Ester werden in Arzneimitteln zur dermalen Anwendung in Konzentrationen von 1 bis 20 Gew.-%, vorzugsweise 5 bis 10 Gew.-%, eingesetzt. Als dermale Formulierungen sind geeignet Salben und Cremes vom Typ Öl in Wasser (O/W) oder Wasser in Öl (W/O) unter Verwendung von üblichen Salgengrundlagen und Hilfsstoffen, wie Fettkompo nenten, Emulgatoren und Stabilisatoren, ferner Gele unter Verwendung von Tylose, Carboxymethylcellulose, Salze von Acrylsäurepolymerisaten oder Acrylsäure-Acrylamid Copolymerisaten als Strukturbildner in wäßrigen oder alkoholischwäßrigen Zubereitungsformen, sowie Lösungen in dermal verträglichen Lösungsmitteln, wie Alkohol, Alkohol-Wasser-Mischungen oder Fettalkoholen und -estern.

Ebenfalls geeignet sind dermale Formulierungen, die zusätzlich Penetrationsvermittler enthalten, wie Dimethylsulfoxid in Konzentrationen von 10 bis 40 Gew.-% oder Harnstoff mit 5 bis 10 Gew.-%.

Die so hergestellten Arzneimittel, insbesondere zur dermalen Applikation werden zur Bekämpfung von Krankheiten des rheumatischen Formenkreises verwendet. Hierbei werden die Diethylenglykolmonoester von Lonazolac und Diclofenac besonders bevorzugt.

Beispiel 1

2-(2-Hydroxyethoxy)-ethyl-[2-(2,6-dichloranilino)-phenylacetat]

291 g (1 Mol) Diclofenac werden in 1110 g (10,5 Mol) Diethylenglykol suspendiert und nach Zugabe von 17,5 g (0,9 Mol) p-Toluolsulfonsäure Monohydrat und 13,4 g (0,115 Mol) o-Phosphorsäure unter Rühren 1 Stunde auf 80 °C erwärmt. Nach dem Abkühlen wird mit einer Lösung von 35 g Natriumcarbonat in 1 Liter Wasser neutralisiert. Der ausgefallene Rohester wird mit zweimal 250 ml Chloroform extrahiert. Die Chloroformphase wird mit Wasser gewaschen, über Natriumsulfat getrocknet und eingedampft. Der Rückstand wird mit einer Mischung von Essigester und Petrolether (2:3, v:v) als Solvens über Kieselgel 60 mittels Mitteldruck-Flüssigkeitschromatographie gereinigt. Nach dem Eindampfen des Lösungsmittels werden 280 g (72 %) eines leicht gelblichen Öles erhalten.

Beispiel 2

2-(2-Hydroxyethoxy)-ethyl-[3-(4-chlorphenyl)-1-phenyl-1H-pyrazol-4-acetat]

3

313 g (1 Mol) Lonazolac werden zu einer Lösung von 30 g (0,29 Mol) konz. Schwefelsäure in 1110 g (10,5 Mol) Diethylenglykol zugegeben und 2 Stunden auf 80 °C erwärmt. Nach dem Abkühlen werden unter Rühren 4,5 Liter 5 %-ige Natriumcarbonatlösung zugesetzt und mit 2 x 250 ml Chloroform extrahiert. Die gewaschene und getrocknete Chloroformlösung wird eingedampft. Der erhaltene Rohester wird an einer Kurzweg-Destillationsanlage bei 210 °C/0,8 Pa destilliert, wobei 264 g (65 %) reiner Ester erhalten werden.

Unter Einsatz von jeweils 1 Mol Säure und 10,5 Mol Diethylenglykol wurden gemäß den Angaben in Tab. 2 folgende weitere Umsetzungen durchgeführt:

Tabelle 2

| Säure | Katalysator(Mol) | Reaktions- | | Reinigung | °C/Pa | Endausbeute % |
| | | Zeit(h) | Temp. °C | | | |
|---|---|---|---|---|---|---|
| Lonazolac | p-Toluolsulfonsäure (0,9) + o-Phosphorsäure(0,115) | 3 | 80 | destill. | 210/0,8 | 76 |
| Lonazolac | o-Phosphorsäure(0,77) | 4 | 120 | MFC* | - | 67 |
| Ketoprofen | p-Toluolsulfonsäure(0,9) + o-Phosphorsäure(0,1) | 3 | 80 | destill. | 170/1,06 | 70 |
| Flurbiprofen | p-Toluolsulfonsäure(0,1) | 2 | 80 | destill. | 180/1,33 | 64 |
| Ibuprofen | p-Toluolsulfonsäure(0,1) | 3 | 80 | destill. | 140/3,99 | 71 |
| (+)-Ibuprofen | dto. | 3 | 80 | destill. | 140/3,99 | 69 |
| (-)-Ibuprofen | dto. | 3 | 80 | MFC* | - | 72 |
| Diclofenac | Polyphosphorsäure (26 g) + p-Toluolsulfonsäure (0,18) | 1 | 80 | MFC* | - | 66 |

* Mitteldruck-Flüssigkeitschromatographie entsprechend Beispiel 1

1) $\alpha = + 45°$

2) $\alpha = - 35°$

EP 0 336 147 A2

**Ansprüche**

1. Diethylenglykolmonoester der antirheumatisch wirksamen Säuren aus der Gruppe bestehend aus Lonazolac, Diclofenac, Flurbiprofen, Ibuprofen und Ketoprofen, einschließlich deren optisch aktiven Isomeren.

2. Verfahren zur Herstellung der Diethylenglykolmonoester nach Anspruch 1, dadurch gekennzeichnet, daß man eine antirheumatisch wirksame Säure aus der Gruppe bestehend aus Lonazolac, Diclofenac, Flurbiprofen, Ibuprofen und Ketoprofen sowie deren optisch aktiven Isomeren in an sich bekannter Weise

a) mit Diethylenglykol bei erhöhter Temperatur, gegebenenfalls in einem inerten organischen Lösungsmittel und in Gegenwart eines sauren Katalysators unter Wasserabscheidung verestert, oder

b) mit einem niedermolekularen $C_1$-$C_5$-Alkohol verestert und das Esterzwischenprodukt in Gegenwart eines basischen Katalysators mit Diethylenglykol umestert.

3. Verfahren nach Anspruch 2a) dadurch gekennzeichnet, daß man eine antirheumatisch wirksame Säure aus der Gruppe bestehend aus Lonazolac, Dichlorfenac, Flurbiprofen, Ibuprofen und Ketoprofen sowie deren optisch aktiven Isomeren mit Diethylenglykol bei einer Temperatur von 80 bis 120 °C ohne azeotrope Entfernung des Reaktionswassers verestert, wobei man einen Überschuß von 8-15, vorzugsweise 10,5-12 Mol Diethylenglykol je Mol Säure einsetzt, überschüssiges Diethylenglykol nach Neutralisation mit Alkali- oder Erdalkalihydroxyden oder -carbonaten abdestilliert oder mit Wasser extrahiert und den erhaltenen Rohester destilliert und/oder mittels Mitteldruck-Flüssigkeitschromatographie reinigt.

4. Arzneimittel enthaltend einen Diethylenglykolmonoester nach Anspruch 1 und übliche galenische Hilfsstoffe.

5. Arzneimittel nach Anspruch 4 zur dermalen Applikation.

6. Arzneimittel nach einem der Ansprüche 4 oder 5, in Form von Salben, Gelen, Cremes oder Lösungen enthaltend einen Diethylenglykolmonoester nach Anspruch 1 in einer Konzentration von 1-20 Gew.-%, vorzugsweise 5-10 Gew.-%.

7. Arzneimittel nach Anspruch 5 zusätzlich enthaltend 10-40 Gew.-% Dimethylsulfoxid und gegebenenfalls 5-10 Gew.-% Harnstoff.

8. Arzneimittel nach einem der Ansprüche 4-7, enthaltend als Wirkstoff den Diethylenglykolmonoester von Lonazolac oder Diclofenac.

9. Verwendung der Diethylenglykolmonoester nach Anspruch 1 zur Herstellung von Arzneimitteln zur Bekämpfung von Krankheiten des rheumatischen Formenkreises.

10. Verwendung des Diethylenglykolmonoesters von Lonazolac und Diclofenac zur Herstellung von dermal applizierbaren Arzneimitteln zur Bekämpfung von Krankheiten des rheumatischen Formenkreises.